# EUROPEAN PATENT APPLICATION

(11) **EP 3 667 747 A1**
(43) Date of publication of application: **17.06.2020**
(21) Application number: 18212012.1
(22) Date of filing: 12.12.2018
(51) Int. Cl.: H01L 41/04, A61M 25/00, H01L 41/09, H01L 41/193

(54) **ACTUATOR DEVICE BASED ON AN ELECTROACTIVE MATERIAL**

(71) Applicant: Koninklijke Philips N.V., 5656 AG Eindhoven (NL)
(72) Inventor: PELSSERS, Eduard Gerard Marie, 5656 AE Eindhoven (NL); JOHNSON, Mark Thomas, 5656 AE Eindhoven (NL); VAN DEN ENDE, Daan Anton, 5656 AE Eindhoven (NL)
(74) Representative: de Haan, Poul Erik

(57) **Abstract**

A device comprises an electroactive material actuator which has a first, low stiffness mode and a second, high stiffness mode. In the high stiffness mode, actuation of portions of the actuator oppose each other such that an increase in stiffness results but still with no resulting overall deformation.

## Description

### FIELD OF THE INVENTION

This invention relates to actuator devices which make use of electroactive materials, such as electroactive polymers.

### BACKGROUND OF THE INVENTION

Electroactive polymers (EAP) are an emerging class of materials within the field of electrically responsive materials. EAPs can work as sensors or actuators and can easily be manufactured into various shapes allowing easy integration into a large variety of systems.

Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications.

An EAP device can be used in any application in which a small amount of movement of a component or feature is desired, based on electric actuation. Similarly, the technology can be used for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 20 kHz.

Devices using electroactive polymers can be subdivided into field-driven and ionic-driven materials.

Examples of field-driven EAPs are dielectric elastomers, electrostrictive polymers (such as PVDF based relaxor polymers or polyurethanes) and liquid crystal elastomers (LCE).

Examples of ionic-driven EAPs are conjugated polymers, carbon nanotube (CNT) polymer composites and Ionic Polymer Metal Composites (IPMC).

Field-driven EAPs are actuated by an electric field through direct electromechanical coupling, while the actuation mechanism for ionic EAPs involves the diffusion of ions. Both classes have multiple family members, each having their own advantages and disadvantages.

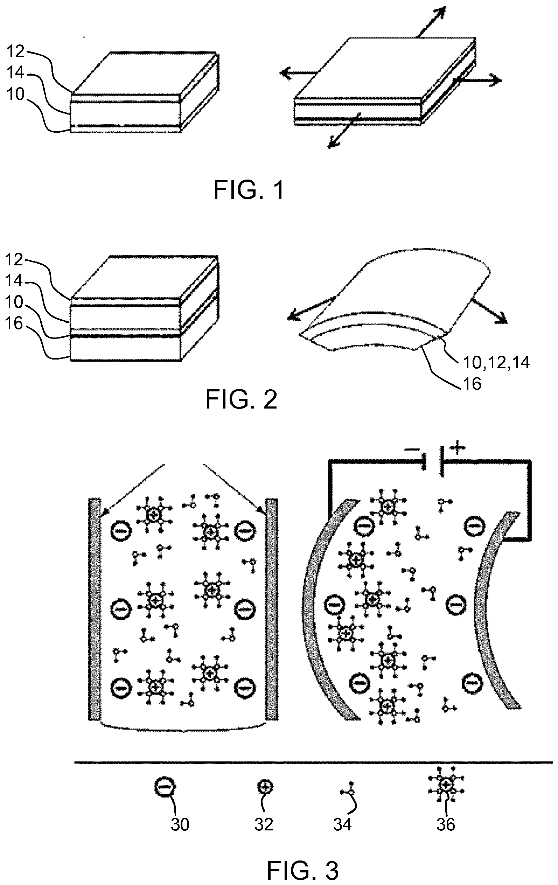

Figures 1 and 2 show two possible operating modes for an EAP device.

The device comprises an electroactive polymer layer 14 sandwiched between electrodes 10, 12 on opposite sides of the electroactive polymer layer 14.

Figure 1 shows a device which is not clamped. A voltage is used to cause the electroactive polymer layer to expand in all directions as shown.

Figure 2 shows a device which is designed so that the expansion arises only in one direction. The device is supported by a carrier layer 16. A voltage is used to cause the electroactive polymer layer to curve or bow.

The nature of this movement for example arises from the interaction between the active layer which expands when actuated, and the passive carrier layer. To obtain the asymmetric curving around an axis as shown, molecular orientation (film stretching) may for example be applied, forcing the movement in one direction.

The expansion in one direction may result from the asymmetry in the electroactive polymer, or it may result from asymmetry in the properties of the carrier layer, or a combination of both.

The electrodes in Figures 1 and 2 for example create an electric field for a field-driven device. Figure 3 shows an example of a current driven ionic device. The actuation mechanism involves the diffusion of ions and/or electrochemical oxidation and reduction. Figure 3 shows the structure of an Ionic Polymer Metal Composite (IPMC). There are fixed anions 30, movable cations 32 and water molecules 34 which attach to the cations to form hydrated cations. These move in response to an applied actuation signal.

A single two-electrode current-driven ionic device will create a bend when actuated, whereas a single two-electrode field-driven device will only expand in the plane. A field-driven device is thus typically combined with a non-stretchable substrate to provide a bending function.

Indeed, EAP actuators are typically formed as bending actuators. They may for example be clamped at one edge, with the actuator projecting from that edge. The projecting part then bends in response to actuation, and the actuation part is for example the remote tip. A double-clamped arrangement is clamped at opposing edges and is caused to bow in response to actuation. The actuation part is then for example the middle of the structure.

These arrangements give little freedom to design particular modes of operation, for example it is difficult to tune the stiffness or the speed of operation in a simple way. The ability to change stiffness of a device on demand is of interest for example in diagnostic and interventional devices that have to travel through tortuous vessels.

US 2016/0352259 discloses a high speed actuator in which a low stiffness is provided to enable high speed actuation. In one example, the stiffness may be controlled by applying a current to a shape memory alloy material to reduce the electrical resistance and increase stiffness.

There remains a need for simple stiffness control of a device based on electroactive material actuators.

### SUMMARY OF THE INVENTION

There is therefore a need for additional modes of operation of a device which makes use of an electroactive material actuator, in particular so that stiffness can be controlled.

It is an object of the current invention to fulfill the aforementioned need at least partially. This object is achieved at least partially by the invention as defined by the independent claims. The dependent claims provide advantageous embodiments.

According to examples in accordance with an aspect of the invention, there is provided a device comprising:
an electroactive material actuator comprising at least first and second portions;
a controller, wherein the controller is adapted to implement at least a first, low stiffness mode and a second, high stiffness mode,
wherein in the first mode the actuator is not operated with no resulting bending deformation of the device; and
wherein in the second mode the actuator is operated, wherein the actuation of the at least first and second portions of the actuator oppose each other such that an increase in stiffness results compared to the first mode but still with no resulting bending deformation.

This device makes use of multiple actuator portions. When operated, they try to deform, but the arrangement of the device is such that these deformations oppose each other, with the result that there is no overall bending deformation but there is an increase in stiffness. Note that by "no overall bending" is meant that any induced bending is negligible compared to an amount of bending that could be induced by a single actuator portion, i.e. without the symmetric cancelling effect.

The rest state does not necessarily need to be flat. Thus, the reference to bending is with reference to the rest state, which may itself be curved.

The design makes use of symmetry in the actuator design to enable a miniature device with controllable stiffness. The device may be used to control the stiffness of a shaft, for example, to assist in steering or to provide rigidity when steering is not desired. The actuation may result in a thickness change to the material layer, but the actuator maintains the same shape in the a length or width direction.

It is an advantage that the stiffness reduces to a minimum when the actuator is not controlled. Thus, in the event of an electrical failure, the device reverts to the lowest stiffness, which may for example be needed to remove the device from a channel in which it is located.

In a first set of examples, the actuator is cylindrical with a first radially inner central actuation electrode and a second radially outer actuation electrode, wherein the first and second portions comprise diametrically opposing portions of the actuator.

In this way, a single actuator may be used, and the different portions are different regions of that actuator. Diametrically opposing regions will try to deform in opposite ways (e.g. bend away from the central axis) but these deformations cancel.

There may be only a single inner electrode and a single outer electrode. However, a segmented outer electrode may be used so that an additional mode may be provided in which there is bending of the device (for example by actuating only some areas around the circumference).

The device may further comprise a second electroactive material actuator, concentric and radially outside the cylindrical actuator. In this way, the is a central cylindrical actuator and a surrounding annular actuator. By controlling the stiffness in both actuators, multiple overall stiffness levels may be set.

The device may comprise a set of three or more electroactive material actuators forming a concentric array, where each electroactive material actuator is independently controllable. Thus gives finer control of the overall stiffness level. The controller may be adapted to actuate a set of actuators to provide stiffness control to a selected one of a set of values.

In a second set of examples, the first and second electroactive material actuator portions are planar in a rest state. In this case, the actuator is planar, for example formed as a flat bending actuator. The two portions are back to back so that attempted bending of the two portions is cancelled when both are actuated. The two portions preferably have separate electrode arrangements although there may be a shared common electrode.

An interface layer is for example between the first and second actuator portions. This may be used to select the range of stiffness values over which adjustment is possible.

The controller may be adapted to implement a third mode in which only one of the first and second actuator portions is controlled such that there is a resulting deformation. Thus the device may implement both stiffness control and bending actuation.

The controller may be adapted to a implement a fourth mode in which the first and second actuator portions are controlled to attempt to deform in an opposite way with respect to the interface layer by different amounts, such that an increase in stiffness results as well as a resulting deformation.

In this way, the device may be used to perform bending as well as stiffness control with independent control of the two functions. A difference in actuation level for example correlates to the resulting bending (so that if the actuation is the same, there is no bending), whereas the common actuation baseline correlates to the stiffness level.

The electroactive material actuator (or each of them, if there is a plurality) may comprise a current-driven actuator such as an ionic polymer metal composite actuator. Alternatively, the electroactive material actuator (or each of them, if there is a plurality) may comprise a field-driven actuator.

Thus, the approach of the invention may be applied with different actuator types. With field driven actuators, large strains can be obtained while ionic actuators can be actuated by low voltages, which can be regarded as a benefit for applications within the human body.

The invention also provides an interventional medical device comprising an intervention shaft comprising a device as defined above, for controlling a stiffness of the intervention shaft. The intervention shaft may comprise a catheter or guidewire, wherein the device is for controlling the stiffness of at least a tip of the intervention shaft.

By controlling the stiffness of the tip of a catheter or guidewire, the insertion process may be improved, by allowing or preventing bending.

The device may be for providing controllable stiffness at a plurality of positions along the intervention shaft. For example, it may be desired to have a rigid tip and a flexible shaft to navigate along a channel.

The invention also provides a method of controlling the stiffness of a device which comprises an electroactive material actuator, the method comprising implementing at least a first, low stiffness mode and a second, high stiffness mode,
wherein the method comprises:
in the first mode not operating the actuator thereby giving no resulting bending deformation of the device; and
in the second mode, controlling the actuator such that the actuation of at least first and second portions of the actuator oppose each other such that an increase in stiffness results compared to the first mode but still with no resulting bending deformation of the device.

### BRIEF DESCRIPTION OF THE DRAWINGS

Examples of the invention will now be described in detail with reference to the accompanying drawings, in which:
Figure 1 shows a known electroactive polymer device which is not clamped;
Figure 2 shows a known electroactive polymer device which is constrained by a backing layer;
Figure 3 shows a current driven electroactive polymer device;

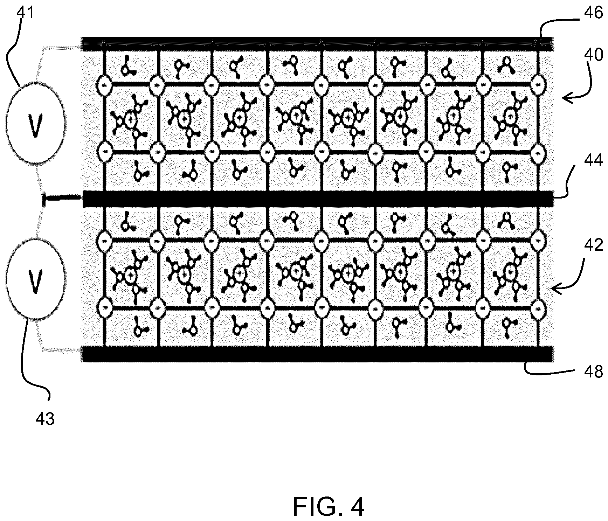

Figure 4 shows a first example of an electroactive material actuator device;

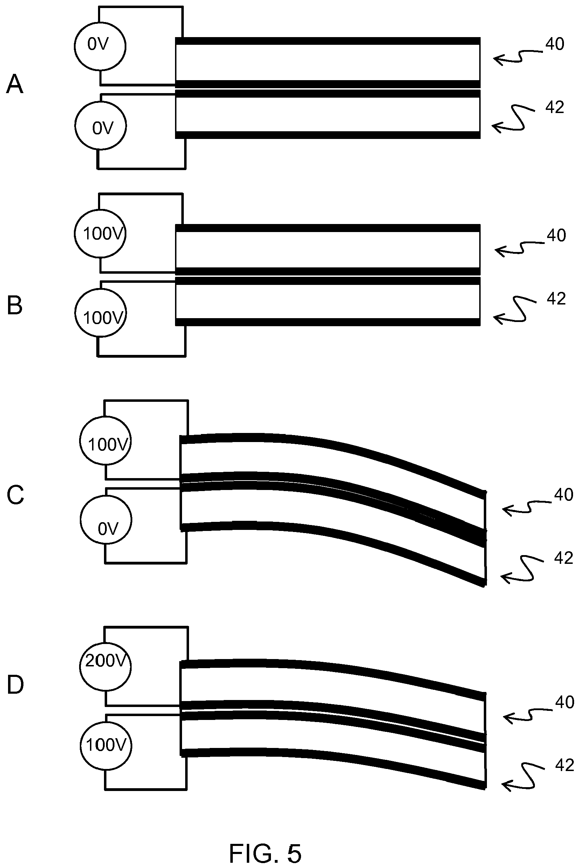

Figure 5 is used to show possible modes of operation of the device of Figure 4;

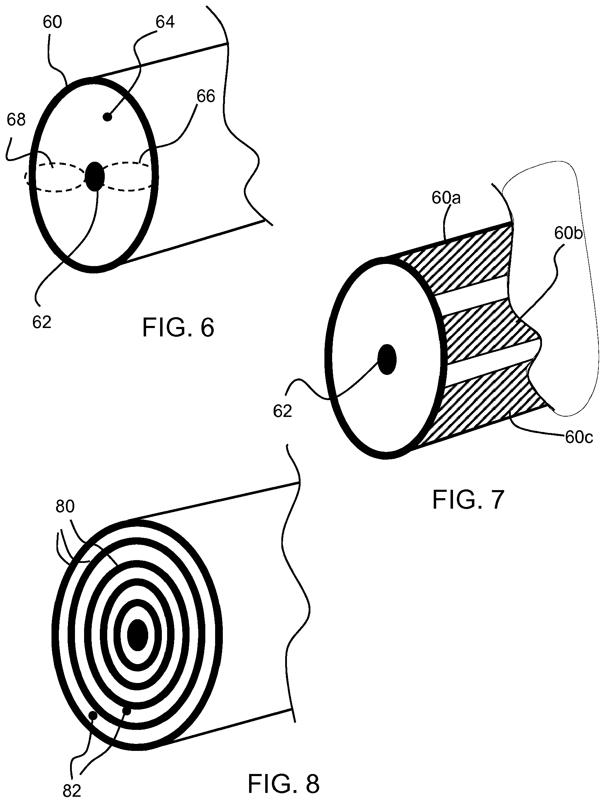

Figure 6 shows a second example of an electroactive material actuator device;
Figure 7 shows a third example of an electroactive material actuator device;
Figure 8 shows a fourth example of an electroactive material actuator device;

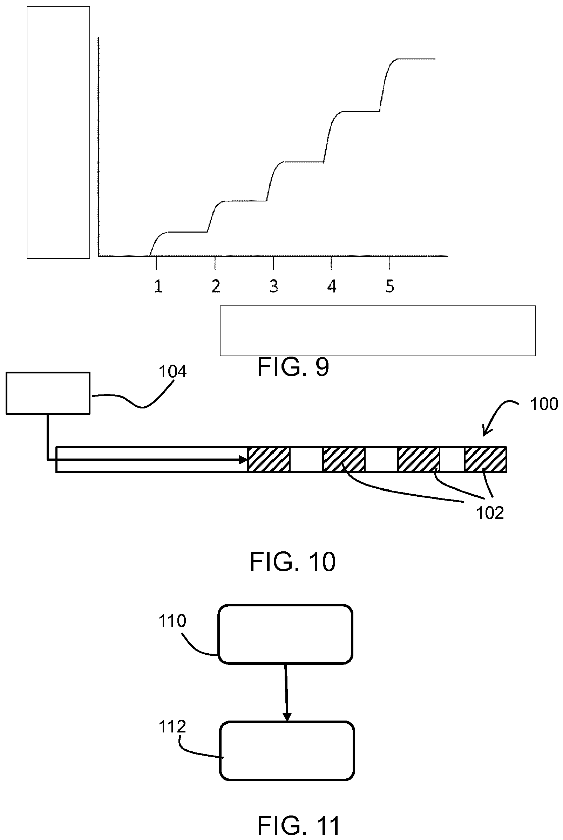

Figure 9 shows how the device of Figure 8 enables stiffness control to multiple levels;
Figure 10 shows a interventional medical device using the actuator; and
Figure 11 shows a stiffness control method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device comprising an electroactive material actuator which has a first, low stiffness mode and a second, high stiffness mode. In the high stiffness mode, actuation of portions of the actuator oppose each other such that an increase in stiffness results but still with no resulting overall bending deformation.

Note that the term "increase in stiffness" is used to denote an increase in the resistance to bending by an external force.

Figure 4 shows a first example, which comprises two ionic EAP actuator portions 40, 42. There is a central electrode 44 which is associated with both of the actuator portions and a respective outer electrode 46, 48 for each actuator portion. Each actuator portion has a voltage source 41, 43 for delivering a voltage between the two electrodes associated with that actuator.

The two portions may be considered to be portions of an overall actuator, although they may in some examples be controlled independently.

This forms a bi-layered actuator which comprises three electrodes and the ionic EAP layer between the two pairs. When the outer electrodes 46, 48 are both set to a negative voltage with respect to the central electrode 44, as can be easily achieved with a DC power source, both the upper and lower actuator structures are activated.

Due to the electrical field between the electrodes, the mobile positive ions will migrate to the outer electrodes and thereby the ionic EAP layer located close to the outer electrodes will increase in density and will increase in stiffness.

With a one-layered structure the actuator would bend, but in the bi-layer structure this is prohibited and consequently the stiffness increases. At the moment the charge on the electrodes is removed, the electrical field is removed and the mobile positive ions will redistribute in a homogenous fashion and the stiffness decreases to its original low value. Hence the structure has a variable stiffness.

In a first mode, the actuator is not operated (i.e. no drive signal is provided to either actuator portion) with no resulting bending deformation of the device (because it is at its rest state) but a low stiffness.

In a second mode the actuator is operated, in particular with equal drive signals to the two actuator portions. The instructed actuation of the first and second portions of the actuator attempts to induce deformations which oppose each other, such that an increase in stiffness results compared to the first mode but still with no resulting bending deformation compared to the rest state.

The stiffness control for example improves the accessibility of small tortuous channels such as small tortuous blood vessels.

The design of Figure 4 may also be formed using a field-driven electroactive polymer. By constraining the device so that it cannot expand in length, each EAP actuator portion will try to bend. Again, the attempted bending of the two actuator portions cancel each other resulting only in a stiffness change.

An interface layer may be provided between the first and second actuator portions 40, 42 to provide the constraint against expansion in the length direction as mentioned above.

Instead of providing an interface layer in the center of the device, two substrates could be introduced on the outer layers. The same symmetrical function is achieved of two actuator portions which attempt to bend in opposing directions.

The bi-layer design may also be used to enable bending control as well as stiffness control. For controlling the bending, the interface layer for example has a high resistance to stretching.

Figure 5 shows possible modes of operation.

Taking the example of a field-driven actuator, when no voltage is applied the device has little resistance against bending which thus defines a low stiffness first mode of operation, shown in Figure 5A.

When a voltage is applied to the actuator portions on both sides of the interface layer, each actuator portion would like to bend but since the two sides provide bending moments in opposing directions (with respect to the boundary between the two portions) this cannot occur (or only in a limited fashion). Consequently a mechanical stress is build up in the device.

When an external force is applied to bend the device, additional stresses build up which will resist bending, hence the device has an increased stiffness.

This defines the second, high stiffness mode, as shown in Figure 5B.

This device can also be brought into a bent configuration by applying a voltage only to one of the actuator portions, as shown in Figure 5C. This provides a first degree of bending, and a corresponding stiffness. This may be considered to be a third mode.

If a voltage is applied to both actuator portions but of different values the device will also bend but since the stiffness is increased, the bend is smaller as shown in Figure 5D.

Thus, a bend with variable stiffness may be provided.

In Figures 5C and 5D, the voltage difference placed over the actuator portions is the same (100V in the example shown), but since the lower actuator is actuated by 100 V in Figure 5D, additional stress is initiated in the device, hence increasing stiffness. Consequently the bend level is lower. Due to the applied voltages the thickness of the layers will decrease somewhat, not shown in the schematic figures.

Figure 6 shows a cylindrical design in which the two actuator portions are formed as a single layer of the electroactive material.

The cylindrical actuator comprises two electrodes 60, 62 and between the electrodes the actuator material 64. The example of an ionic EAP will first be described. There is an annular outer electrode 60 and a central rod electrode 62.

The single actuator may be considered to comprise multiple portions, such as a first portion 66 and a diametrically opposed second portion 68. The deformation attempted by these two portions cancel when the overall actuator is operated using the outer electrode 60 which surrounds the full actuator.

At the limit, the single actuator may be considered to comprise an infinite number of infinitesimal portions in diametrically opposed pairs.

For an ionic actuator material, when the outer electrode 60 is set to a negative voltage with respect to the central electrode 62, as can be easily achieved with a DC power source, the actuator is activated. Due to the electrical field between the electrodes, the mobile positive ions will migrate to the outer electrode and thereby the ionic EAP located close to the outer electrode will increase in density and will increase the stiffness but no bending can occur.

Due to the cylindrical symmetric shape, the actuator will not bend. At the moment the charge on the electrodes is removed, the electrical field is removed and the mobile positive ions will redistribute in a homogenous fashion and the stiffness decreases to its original low value.

The ionic device may be made to increase in stiffness when positive ions move radially out (as explained for the example above) or when positive ions move radially in. However, the effect is larger with the positive ions migrating to outer rim.

When a cylinder is made to bend by an external force, at the inner radius of the bend the material must be squeezed, at the center line there is no expansion or squeezing and at the outer radius the material has to expand. Thus, by moving positive ions towards the outer edge, they are increasing the resistance to bending where it is needed to have most effect, i.e. including regions where an increased resistance to squeezing will increase the resistance to an external bending force.

The example of Figure 6 may also be implemented using a field-driven electroactive polymer actuator. When a field-driven device is used to form the structure shown in Figure 6, actuation will not lead to a migration. In fact an attraction will occur between the electrodes that will symmetrically try to squeeze the electroactive polymer and hence an expansion over the length will occur, but no bending.

Only when an almost non-stretchable substrate is added to the inner part or the outer part, this expansion can no longer occur and hence again only an increase in stiffness is obtained.

Thus, a substrate may be provided at the center which has a high resistance against expansion. Alternatively, instead of introducing a substrate in the center of the device, a cylindrical substrate could be introduced on the outer layer of the device, again obtaining a symmetrical device where variable stiffness can be introduced by applying a voltage. Note that in Figure 6, at the ends of the cylinder (one of which is depicted in Figure 6) there may be some expansion, but this only affects the actuator locally at the edges.

There are thus differences between the ionic EAP implementation and the field driven EAP implementation. Within an ionic device, there is a volume displacement within the actuator material (because ions migrate) while with a field-driven device there is no volume displacement within the actuator material (dipoles are formed but are fixed in the polymer matrix).

The example of Figure 6 has two continuous electrodes. Figure 7 shows an example in which the outer electrode 60 is segmented into regions 60a, 60b, 60c at different circumferential locations. If all electrodes are operated simultaneously, the same function arises as for Figure 6. However, by operating only one or more selected outer electrode regions, bending can be induced. Each electrode region may then be considered to be associated with its own electroactive polymer actuator portion.

Figure 8 shows an example in which the cylindrical design is extended to a multi-layered actuator structure with a plurality of concentric electrodes 80 and between the electrodes concentric annular regions 82 of the electroactive material, such as the ionic EAP material. When an outer electrode is set on a negative voltage with respect to the previous inner electrode, as can be easily achieved with a DC power source, the actuator layer between them is activated. Due to the electrical field between the electrodes, the mobile positive ions will migrate to the outer electrode and thereby the ionic EAP located close to the outer electrode will increase in density and will increase the stiffness.

The actuator layers can be selectively activated. By stepwise activation of the layers, the stiffness can be varied in a stepwise manner. By changing the DC voltage across the different layers, the stiffness can be varied to more levels than can be achieved simply based on the binary selection of actuated layers.

For best accuracy, the various actuator layers can be driven by a DC voltage to a saturation level where the stiffness change is more precise. In his way, the structure of Figure 8 enables a precise stepwise change in stiffness, as schematically shown in Figure 9, which shows a stiffness (arbitrary unit) on the y-axis versus a number of actuated annular actuator portions on the x-axis.

In this example, a five layered actuator is employed, hence a 5 step activation leads to a five step variation in stiffness.

This multi-layered cylindrical design may also be applied with a field-driven EAP actuator.

The invention is of particular interest for interventional medical devices (IMD's) such as catheters and guidewires.

Figure 10 shows an interventional device comprising a shaft 100 having regions 102 where the stiffness can be controlled. A controller 104 provides the control signals for driving the actuators in the regions 102.

The shaft 100 needs to have a small diameter (for example between 1.5 and 10mm for a catheter, and between 0.5mm and 1.5mm for a guidewire) to be able to penetrate into the smaller blood vessels. Even then, traversing through tortuous blood vessels is still a challenge. This is especially a problem when the device is traversed in a bend, for example when the device is fed into a leg of a bifurcation of small diameter. When traversing into such a bend, a low stiffness is desired because when the device is stiff it can resist bending and thereby resist further feeding into the bend of a blood vessel. When the catheter tip bends around a corner, it is desired that the segment just behind the tip is temporarily compliant such that the rest of the catheter follows the tip.

An IMD with low stiffness over its complete length is not desirable, this would impact on the ability to push the IMD. In addition, when the device is required to penetrate a blockage, for instance a chronic total occlusion, a relatively stiff device is desired when attempting to cross the occlusion.

Thus, a variable stiffness over the length of the shaft, or partially over the length of the shaft, is in demand for an IMD. Preferably, the tip part of the IMD that is at the position of a bend should have a low stiffness to be able to follow the bend in the blood vessel without kicking or without piercing the blood vessel. Also, when the IMD is at the desired position, for instance when the tip of the device reaches an occlusion, the device should be stiff over its complete length to be able to cross the occlusion.

The part of the IMD that is at a bend will change over time by virtue of feeding of the IMD into the blood vessel system, hence it may be beneficial that the stiffness can be controlled as function of the length of the IMD. For instance, when entering a progressively smaller blood vessel system it may be desired to implement a gradual decrease in stiffness, hence the IMD part still in a wide blood vessel should have a stiffer value then the IMD part already in a blood vessel with smaller diameter and/or higher tortuous level.

In addition to stiffness control, actuators along a catheter or guidewire shaft may also be used for active steering, as is known in the art.

Figure 11 shows a method of controlling the stiffness of a device which comprises an electroactive material actuator, the method comprising:
in step 110, implementing a low stiffness mode in which the actuator is not operated thereby giving no resulting bending deformation of the device; and
in step 112 implementing a high stiffness mode by operating the actuator such that the actuation of at least first and second portions of the actuator oppose each other such that an increase in stiffness results compared to the low stiffness mode but still with no resulting bending deformation of the device.

In all examples, the electroactive material actuator is typically based on an electroactive polymer material, although the invention can in fact be used for devices based on other kinds of EAM material. Such other EAM materials are known in the art and the person skilled in the art will know where to find them and how to apply them. A number of options will be described herein below.

A common sub-division of EAM devices is into field-driven and current or charge (ion) driven EAMs. Field-driven EAMs are actuated by an electric field through direct electromechanical coupling, while the actuation mechanism for current or charge driven EAMs involves the diffusion of ions. The latter mechanism is more often found in the corresponding organic EAMs such as EAPs. While field driven EAMs generally are driven with voltage signals and require corresponding voltage drivers/controllers, current driven EAMs generally are driven with current or charge signals sometimes requiring current drivers. Both classes of materials have multiple family members, each having their own advantages and disadvantages.

Field driven EAMs can be organic or inorganic materials and if organic can be single molecule, oligomeric or polymeric. For the current invention they are preferably organic and then also oligomeric or even polymeric. The organic materials and especially polymers are an emerging class of materials of growing interest as they combine the actuation properties with material properties such as light weight, cheap manufacture and easy processing.

The field driven EAMs and thus also EAPs are generally piezoelectric and possibly ferroelectric and thus comprise a spontaneous permanent polarization (dipole moment). Alternatively, they are electrostrictive and thus comprise only a polarization (dipole moment) when driven, but not when not driven. Alternatively they are dielectric relaxor materials. Such polymers include, but are not limited to, the sub-classes: piezoelectric polymers, ferroelectric polymers, electrostrictive polymers, relaxor ferroelectric polymers (such as PVDF based relaxor polymers or polyurethanes), dielectric elastomers, liquid crystal elastomers. Other examples include electrostrictive graft polymers, electrostrictive paper, electrets, electroviscoelastic elastomers and liquid crystal elastomers.

The lack of a spontaneous polarization means that electrostrictive polymers display little or no hysteretic loss even at very high frequencies of operation. The advantages are however gained at the expense of temperature stability. Relaxors operate best in situations where the temperature can be stabilized to within approximately 10 °C. This may seem extremely limiting at first glance, but given that electrostrictors excel at high frequencies and very low driving fields, then the applications tend to be in specialized micro actuators. Temperature stabilization of such small devices is relatively simple and often presents only a minor problem in the overall design and development process.

Relaxor ferroelectric materials can have an electrostrictive constant that is high enough for good practical use, i.e. advantageous for simultaneous sensing and actuation functions. Relaxor ferroelectric materials are non-ferroelectric when zero driving field (i.e. voltage) is applied to them, but become ferroelectric during driving. Hence there is no electromechanical coupling present in the material at non-driving. The electromechanical coupling becomes non-zero when a drive signal is applied and can be measured through applying the small amplitude high frequency signal on top of the drive signal. Relaxor ferroelectric materials, moreover, benefit from a unique combination of high electromechanical coupling at non-zero drive signal and good actuation characteristics.

The most commonly used examples of inorganic relaxor ferroelectric materials are: lead magnesium niobate (PMN), lead magnesium niobate-lead titanate (PMN-PT) and lead lanthanum zirconate titanate (PLZT). But others are known in the art.

PVDF based relaxor ferroelectric based polymers show spontaneous electric polarization and they can be pre-strained for improved performance in the strained direction. They can be any one chosen from the group of materials herein below.

Polyvinylidene fluoride (PVDF), Polyvinylidene fluoride - trifluoroethylene (PVDF-TrFE), Polyvinylidene fluoride - trifluoroethylene - chlorofluoroethylene (PVDF-TrFE-CFE), Polyvinylidene fluoride - trifluoroethylene - chlorotrifluoroethylene) (PVDF-TrFE-CTFE), Polyvinylidene fluoride- hexafluoropropylene (PVDF - HFP), polyurethanes or blends thereof.

The sub-class dielectric elastomers includes, but is not limited to: acrylates, polyurethanes, silicones.

Examples of ionic-driven EAPs are conjugated polymers, carbon nanotube (CNT) polymer composites and Ionic Polymer Metal Composites (IPMC).

The sub-class conjugated polymers includes, but is not limited to:
polypyrrole, poly-3,4-ethylenedioxythiophene, poly(p-phenylene sulfide), polyanilines.

The materials above can be implanted as pure materials or as materials suspended in matrix materials. Matrix materials can comprise polymers.

To any actuation structure comprising EAM material, additional passive layers may be provided for influencing the behavior of the EAM layer in response to an applied drive signal.

The actuation arrangement or structure of an EAM device can have one or more electrodes for providing the control signal or drive signal to at least a part of the electroactive material. Preferably the arrangement comprises two electrodes. The EAM layer may be sandwiched between two or more electrodes. This sandwiching is needed for an actuator arrangement that comprises an elastomeric dielectric material, as its actuation is among others due to compressive force exerted by the electrodes attracting each other due to a drive signal. The two or more electrodes can also be embedded in the elastomeric dielectric material. Electrodes can be patterned or not.

It is also possible to provide an electrode layer on one side only for example using interdigitated comb electrodes.

A substrate can be part of the actuation arrangement. It can be attached to the ensemble of EAP and electrodes between the electrodes or to one of the electrodes on the outside.

The electrodes may be stretchable so that they follow the deformation of the EAM material layer. This is especially advantageous for EAP materials. Materials suitable for the electrodes are also known, and may for example be selected from the group consisting of thin metal films, such as gold, copper, or aluminum or organic conductors such as carbon black, carbon nanotubes, graphene, poly-aniline (PANI), poly(3,4-ethylenedioxythiophene) (PEDOT), e.g. poly(3,4-ethylenedioxythiophene) poly(styrenesulfonate) (PEDOT:PSS). Metalized polyester films may also be used, such as metalized polyethylene terephthalate (PET), for example using an aluminum coating.

The materials for the different layers will be selected for example taking account of the elastic moduli (Young's moduli) of the different layers.

Additional layers to those discussed above may be used to adapt the electrical or mechanical behavior of the device, such as additional polymer layers.

An electroactive polymer structure as described above may be used for actuation and/or for sensing. The most prominent sensing mechanisms are based on force measurements and strain detection. Dielectric elastomers, for example, can be easily stretched by an external force. By putting a low voltage on the sensor, the strain can be measured as a function of voltage (the voltage is a function of the area).

Another way of sensing with field driven systems is measuring the capacitance-change directly or measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. O₂, NO₂), making CNTs usable as gas detectors.

There are many uses for electroactive material actuators and sensors. In many applications the main function of the product relies on the (local) manipulation of human tissue, or the actuation of tissue contacting interfaces. In such applications EAP actuators provide unique benefits mainly because of the small form factor, the flexibility and the high energy density. Hence EAPs can be easily integrated in soft, 3D shaped and / or miniature products and interfaces. Examples of such applications are:
Skin cosmetic treatments such as skin actuation devices in the form of EAP based skin patches which apply a constant or cyclic stretch to the skin in order to tension the skin or to reduce wrinkles;
Respiratory devices with a patient interface mask which has an EAP based active cushion or seal, to provide an alternating normal pressure to the skin which reduces or prevents facial red marks;
Electric shavers with an adaptive shaving head. The height of the skin contacting surfaces can be adjusted using EAP actuators in order to influence the balance between closeness and irritation;
Oral cleaning devices such as an air floss with a dynamic nozzle actuator to improve the reach of the spray, especially in the spaces between the teeth. Alternatively, toothbrushes may be provided with activated tufts;
Consumer electronics devices or touch panels which provide local haptic feedback via an array of EAP transducers which is integrated in or near the user interface;
Catheters with a steerable tip to enable easy navigation in tortuous blood vessels. The actuator function for example controls the bending radius to implement steering, as explained above.

Another category of relevant application which benefits from EAP actuators relates to the modification of light. Optical elements such as lenses, reflective surfaces, gratings etc. can be made adaptive by shape or position adaptation using EAP actuators. Here the benefits of EAP actuators are for example the lower power consumption.

Some examples where stiffness control is of particular interest are outlined below.

Actuators may be used in valves, including human implantable devices such as prosthetic heart valves or valves in organ-on-chip applications or microfluidic devices. For many valves, an asymmetric behavior is desired: compliant and large displacement in a direction with the flow, and stiff in a direction against the flow. Sometimes high actuation speed is required to close a valve quickly.

A flexible display actuator is desired in some applications, for example in smart bracelets. When the flexible display moves to another position or shape for better reading or visual performance, a large displacement is required. When the display is in its rest position, the display actuator must be stiff to hold its position firmly.

There are also application is noise and vibration control systems. Using stiffness variation, it is possible to move away from resonance frequencies and hence reduce vibrations. This is useful for example in in surgery robotic tools where precision is important.

Soft robotics (artificial muscle systems supporting the human body) for example is used to support or hold a body part in a certain position (e.g. against gravity), during which stiffness is required. When the body part moves in the opposite direction resistance is not required and low stiffness is desirable.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. A device comprising:
an electroactive material actuator comprising at least first and second portions (40,42; 66,68);
a controller (104), wherein the controller is adapted to implement at least a first, low stiffness mode and a second, high stiffness mode,
wherein in the first mode the actuator is not operated with no resulting bending deformation of the device; and
wherein in the second mode the actuator is operated, wherein the actuation of the at least first and second portions of the actuator oppose each other such that an increase in stiffness results compared to the first mode but still with no resulting bending deformation.

2. A device as claimed in claim 1, wherein the actuator is cylindrical with a first radially inner central actuation electrode (62) and a second radially outer actuation electrode (60), wherein the first and second portions (66.68) comprise diametrically opposing portions of the actuator.

3. A device as claimed in claim 1 or 2, further comprising a second electroactive material actuator, concentric and radially outside the cylindrical actuator.

4. A device as claimed in any one of claims 1 to 3, comprising a set of three or more electroactive material actuators (82) forming a concentric array, where each electroactive material actuator is independently controllable.

5. A device as claimed in claim 4, wherein the controller is adapted to actuate a set of actuators to provide stiffness control to a selected one of a set of values.

6. A device as claimed in claim 1, wherein the first and second portions (40, 42) are planar in a rest state.

7. A device as claimed in claim 6, further comprising an interface layer between the first and second actuator portions.

8. A device as claimed in claim 6 or 7, wherein the controller is adapted to implement:
a third mode in which only one of the first and second actuator portions (40, 42) is controlled such that there is a resulting deformation; or
a fourth mode in which the first and second actuator portions (40, 42) are controlled to attempt to deform in an opposite way with respect to the interface layer by different amounts, such that an increase in stiffness results as well as a resulting deformation.

9. A device as claimed in any preceding claim, wherein the or each electroactive material actuator comprises a current-driven actuator.

10. A device as claimed in claim 9, wherein the or each electroactive material actuator comprises an ionic polymer metal composite actuator.

11. A device as claimed in any one of claims 1 to 8, wherein the or each electroactive material actuator comprises a field-driven actuator.

12. An interventional medical device comprising an intervention shaft (100) comprising a device as claimed in any one of claims 1 to 11, for controlling a stiffness of the intervention shaft.

13. An interventional medical device as claimed in claim 12, wherein the intervention shaft comprises a catheter or guidewire, wherein the device is for controlling the stiffness of at least a tip of the intervention shaft.

14. An interventional medical device as claimed in claim 13, wherein the device is for providing controllable stiffness at a plurality of positions (102) along the intervention shaft.

15. A method of controlling the stiffness of a device which comprises an electroactive material actuator, the method comprising implementing at least a first, low stiffness mode and a second, high stiffness mode,
wherein the method comprises:
(110) in the first mode not operating the actuator thereby giving no resulting bending deformation of the device; and
(112) in the second mode, controlling the actuator such that the actuation of at least first and second portions of the actuator oppose each other such that an increase in stiffness results compared to the first mode but still with no resulting bending deformation of the device.
